# EUROPEAN PATENT APPLICATION

(11) **EP 0 559 285 A1**
(43) Date of publication of application: **08.09.1993**
(21) Application number: 93200570.5
(22) Date of filing: 02.03.1993
(51) Int. Cl.: C07D 491/056, A61K 31/44

(54) **1,4-Dioxino 2,3-b pyridine derivatives having serotonergic activity**

(30) Priority: 06.03.1992 EP 92400603
(71) Applicant: Akzo Nobel N.V., NL-6824 BM Arnhem (NL)
(72) Inventor: Guillaumet, Gérald, F-45100 Orleans (FR); Poirot, Pascal, F-54000 Nancy (FR); Benarab, Abdelhakim, F-45100 Orleans la Source (FR)
(74) Representative: Hermans, Franciscus G.M.

(57) **Abstract**

This invention is concerned with 1,4-dioxino[2,3-b]-pyridine derivatives having formula I 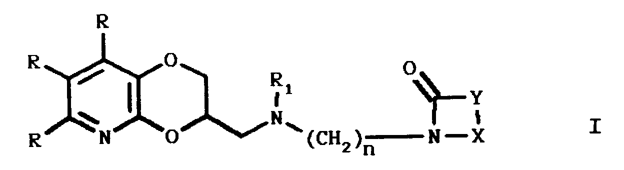
wherein each of the groups R is independently selected from hydrogen, hydroxy, lower alkyl, lower alkoxy, CF₃, and halogen;
R₁ is hydrogen or lower alkyl;
n is 2, 3, or 4;
X is CH₂, C=O, or SO₂; and
Y is substituted or unsubstituted 1,2-phenylene, -(CH₂)₄-, -CH₂CR₂R₃-, or -CH₂CR₂R₃CH₂-, in which R₂ and R₃ are independently hydrogen or lower alkyl, or together 1,4-butanediyl or 1,5-pentanediyl; or pharmaceutically acceptable salts thereof.

The compounds according to the invention are strong serotonin ligands with preference for the 5HT_{1A} receptor and may, therefore, find application as anti-depressants or as a drug in combating anxiety disorders.

## Description

The invention relates to 1,4-dioxino[2,3-b]pyridine derivatives having serotonergic activity. The invention is further concerned with a process for the preparation of said pyridine derivatives, with a pharmaceutical composition containing the same, and with a use thereof for the preparation of a medicament.

Some 1,4-dioxino[2,3-b]pyridine derivatives are known, for example from H. Neunhoeffer and O. Sponheimer (Chem. Ber., 123 (1990), 2453-2454), but these compounds have no functionality at the dioxin moiety and no pharmacological activity is described.
Corresponding derivatives having a benzo[1,4]dioxan moiety are also known. For example, European patent application EP 170,213 discloses glutarimide agents having antianxiety and antihypertensive activity, and European patent application EP 236,930 (Example I) and German patent application DE 3,726,425 (Example 3) disclose 2-[4-[(2,3-dihydro-1,4-benzodioxin-2-yl)methyl-amino]butyl]-1,2-benzisothiazol-3(2H)one-1,1-dioxide.
These compounds, however, although displaying affinity to the 5HT_{1A} receptor, have an unfavourable pharmacological profile in comparison with the compounds of the present invention, which render these known compounds less suitable for administration in humans.

The compounds of this invention are 1,4-dioxino[2,3-b]-pyridine derivatives having formula I 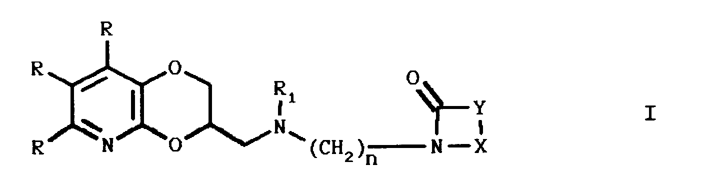
wherein each of the groups R is independently selected from hydrogen, hydroxy, lower alkyl, lower alkoxy, CF₃, and halogen;
R₁ is hydrogen or lower alkyl;
n is 2, 3, or 4;
X is CH₂, C=O, or SO₂; and
Y is substituted or unsubstituted 1,2-phenylene, -(CH₂)₄-, -CH₂CR₂R₃-, or -CH₂CR₂R₃CH₂-, in which R₂ and R₃ are independently hydrogen or lower alkyl, or together 1,4-butanediyl or 1,5-pentanediyl; or pharmaceutically acceptable salts thereof.

The compounds according to the invention are strong serotonin ligands with preference for the 5HT_{1A} receptor. They have a favourable pharmacological profile (as shown, for example, by their favourable ratio for affinity to the 5HT_{1A} receptor with respect to the α₁-adrenoceptor), displaying minimal side-effects like sedation and orthostatic hypotension. The compounds may, therefore, find application as anti-depressants or as a drug in combating anxiety disorders.

The term lower alkyl used in the definition of general formula I, means a branched or unbranched alkyl group having 1-6 carbon atoms, such as methyl, ethyl, propyl, isopropyl, butyl, *tert*-butyl, pentyl, hexyl and the like. Preferred alkyl groups have 1-4 carbon atoms, and the most preferred is the methyl group.

The alkyl moiety which is present in the lower alkoxy group has the same meaning as previously defined for lower alkyl.

The term halogen used in the definition of formula I means fluorine, chlorine, bromine or iodine. Fluorine is the preferred halogen.

The substituents which may be present at the 1,2-phenylene moiety, are selected from hydroxy, lower alkyl, lower alkoxy, CF₃, and halogen.

The novel compounds of formula I may be isolated from the reaction mixture in the form of a pharmaceutically acceptable salt. The pharmaceutically acceptable salts may also be obtained by treating the free base of formula I with an organic or inorganic acid such as HCl, HBr, HI, H₂SO₄, H₃PO₄, acetic acid, propionic acid, glycolic acid, maleic acid, malonic acid, methane-sulphonic acid, fumaric acid, succinic acid, tartaric acid, citric acid, benzoic acid, and ascorbic acid.

The compounds of this invention possess one or more chiral carbon atoms, and may therefore be obtained as a pure enantiomer, or as a mixture of enantiomers, among which the racemic mixture. Methods for obtaining the pure enantiomers are well known in the art, e.g. synthesis with chiral induction, synthesis starting from chiral intermediates, crystallization of salts which are obtained from optically active acids and the racemic mixture, or chromatography using a chiral column.

Preferred compounds according to the invention are the 1,4-dioxino[2,3-b]pyridine derivatives in which R is hydrogen, or pharmaceutically acceptable salts thereof. Other preferred compounds have X is C=O, or pharmaceutically acceptable salts thereof. Most preferred are the 1,4-dioxino[2,3-b]pyridine derivatives in which R₁ is hydrogen, n is 4, X is C=O, Y is -CH₂CR₂R₃CH₂-, and R₂ and R₃ are together 1,4-butanediyl, or pharmaceutically acceptable salts thereof.

The compounds of the invention can be prepared by methods which are in use for the preparation of analogous compounds. A suitable method of preparing the 1,4-dioxino[2,3-b]pyridine derivatives according to the invention is characterized in that
a) a pyridine derivative having the formula II 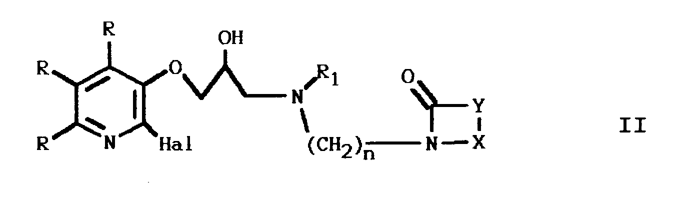 wherein R, R₁, n, X and Y have the previously given meanings, and Hal is Cl, Br, or I, is cyclized, or
b) an amine having formula III 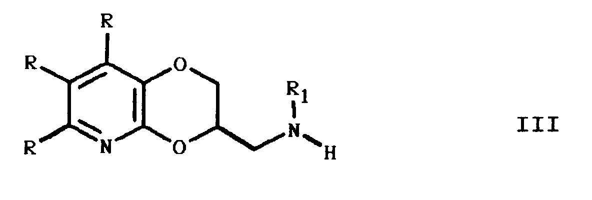 wherein R and R₁ have the previously given meanings, is condensed with a compound having formula IV 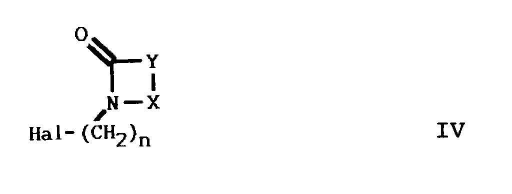 wherein Hal, n, X and Y have the previously given meanings, or
c) a halide having formula V 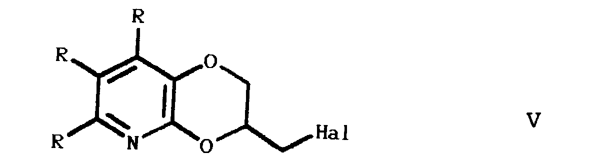 wherein R and Hal have the previously given meanings, is condensed with an amine having formula VI 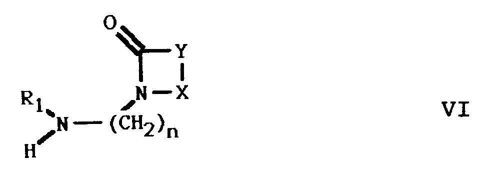 wherein R₁, n, X and Y have the previously given meanings, after which the compound obtained may be separated into its enantiomers and/or converted into a pharmaceutically acceptable salt.

The compounds of the invention may be administered enterally or parenterally, and for humans preferably in a daily dosage of 0,001-10 mg per kg body weight. Mixed with pharmaceutically suitable auxiliaries, e.g. as described in the standard reference, Gennaro et al., Remington's Pharmaceutical Sciences, (18th ed., Mack Publishing Company, 1990, see especially Part 8: Pharmaceutical Preparations and Their Manufacture) the compounds may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, e.g. a nasal spray. For making dosage units, e.g. tablets, the use of conventional additives such as fillers, colorants, polymeric binders and the like is contemplated. In general any pharmaceutically acceptable additive which does not interfere with the function of the active compounds can be used.
Suitable carriers with which the compositions can be administered include lactose, starch, cellulose derivatives and the like, or mixtures thereof, used in suitable amounts.

The invention is further illustrated by the following examples.

### Example 1

### 1,3-dihydro-2-[N-(2,3-dihydro-1,4-dioxino[2,3-b]pyridin-3-ylmethyl)-4-aminobutyl]-1,3-dioxo-2H-isoindole

a. Under an inert atmosphere 3,88 g of 2-chloro-3-pyridinol in 60 ml of dry N,N-dimethylformamide (DMF) were added portionwise at room temperature to 1,58 g of sodium hydride 50%, which were washed with a minimum amount of tetrahydrofuran (THF). Then 27,75 g of epichlorohydrin, dissolved in 50 ml of dry DMF, were added, and the reaction mixture was stirred for 48 h at 60 °C. When the reaction was terminated, the mixture was cooled and hydrolysed with 150 ml of water. The reaction product was extracted into dichloromethane, the organic layer was washed with 5% aqueous sodium carbonate and dried over magnesium sulfate. The solvent was evaporated and the residue was purified by silica chromatography (petroleum ether-diethyl ether 8:2→3:7) to give 5 g (89%) of 2-chloro-3-(oxiranylmethoxy)pyridine. m.p. 34-36 °C.
b. Benzylamine (1,79 g) was added to 1,25 g of 2-chloro-3-(oxiranylmethoxy)pyridine in 30 ml of THF, and the reaction mixture was stirred for 30 h at 60 °C. The mixture was cooled, extracted with dichloromethane, and the organic layer was washed with 5% aqueous sodium carbonate and dried over magnesium sulfate. The solvent was evaporated to give after purification by silica chromatography (dichloromethane-methanol 95:5→90:10) 1,77 g (90%) of 1-(2-chloro-3-pyridinyl-oxy)-3-(phenylmethylamino)-2-propanol. m.p. 65-66 °C.
c. 1-(2-chloro-3-pyridinyloxy)-3-(phenylmethylamino)-2-propanol was cyclized to 3-(2,3-dihydro-N-phenyl-methyl-1,4-dioxino[2,3-b]pyridine)methanamine by one of the following methods:
   (i) 300 mg of 1-(2-chloro-3-pyridinyloxy)-3-(phenyl-methylamino)-2-propanol dissolved in 2 ml of *tert*-butanol was added to a solution of 48 mg of potassium in 10 ml of *tert*-butanol, and the mixture was heated at 70 °C for 20 h. After addition of water the product was extracted with dichloromethane, the organic layer was washed with 5% aqueous sodium hydrogen carbonate and dried over magnesium sulfate. After evaporation to dryness 3-(2,3-dihydro-N-phenylmethyl-1,4-dioxino[2,3-b]-pyridine)methanamine was obtained.
   (ii) 300 mg of 1-(2-chloro-3-pyridinyloxy)-3-(phenyl-methylamino)-2-propanol dissolved in 3 ml of DMF was added to a suspension of 60 mg of 50% sodium hydride in 10 ml of DMF. The mixture was stirred at 95 °C for 24 h to afford 3-(2,3-dihydro-N-phenyl-methyl-1,4-dioxino[2,3-b]pyridine)methanamine.
   (iii) In a manner similar as described in ii 3-(2,3-dihydro-N-phenylmethyl-1,4-dioxino[2,3-b]-pyridine)methanamine was obtained, using sodium hydride in THF at 55 °C.
   (iv) In a manner similar as described in (ii) 3-(2,3-dihydro-N-phenylmethyl-1,4-dioxino[2,3-b]-pyridine)methanamine was obtained, using sodium hydride in a mixture of THF and 10% hexamethyl phosphorous triamide at 55 °C.
   (v) In a manner similar as described in (ii) 3-(2,3-dihydro-N-phenylmethyl-1,4-dioxino[2,3-b]-pyridine)methanamine was obtained, using sodium hydride in dimethyl sulfoxide at 60 C.
   (vi) In a manner similar as described in (ii) 63% of 3-(2,3-dihydro-N-phenylmethyl-1,4-dioxino[2,3-b]-pyridine)methanamine was obtained, using sodium hydride in 1,2-dimethoxyethane at 80 °C.
d. To 3-(2,3-dihydro-N-phenylmethyl-1,4-dioxino[2,3-b]-pyridine)methanamine, dissolved in dry methanol which was acidified using concentrated hydrochloric acid, 10% palladium on charcoal was added. The mixture was stirred under an atmosphere of hydrogen, and after complete hydrogenation the product was filtered, neutralized using potassium carbonate, and purified by column chromatography over silica (dichloromethane-methanol 9:1) to obtain pure 3-(2,3-dihydro-1,4-dioxino[2,3-b]pyridine)methanamine (yield 77%).
e. 200 mg of 3-(2,3-dihydro-1,4-dioxino[2,3-b]pyridine)-methanamine were dissolved in 7 ml of DMF, and 406 mg of (4-bromobutyl)-phthalimide, 363 mg of triethylamine, and 10 mg of potassium iodide were added. The reaction mixture was heated at 60 °C, and monitored by thin layer chromatography. After completion, water was added and the product was extracted with dichloromethane. The organic layer was washed with saturated aqueous sodium hydrogen carbonate, dried over magnesium sulphate, evaporated to dryness, and the residue was purified by column chromatography over silica (dichloromethane-methanol 98:2→90:10) to obtain 229 mg (52%) of 1,3-dihydro-2-[N-(2,3-dihydro-1,4-dioxino[2,3-b]pyridin-3-ylmethyl)-4-aminobutyl]-1,3-dioxo-2H-isoindole. m.p. 80 °C.

### Example 2

In an analogous manner as described in Example 1 were prepared:
1-[N-(2,3-dihydro-1,4-dioxino[2,3-b]pyridin-3-ylmethyl)-4-aminobutyl]-4,4-dimethyl-piperidine-2,6-dione ethanedioate (1:1). m.p. 208 °C.

1-[N-(2,3-dihydro-1,4-dioxino[2,3-b]pyridin-3-ylmethyl)-4-aminoethyl]-4,4-dimethyl-piperidine-2,6-dione (E)-2-butenedioate (1:1).

1,3-dihydro-2-[N-(2,3-dihydro-1,4-dioxino[2,3-b]pyridin-3-ylmethyl)-N-methyl-4-aminobutyl]-1,3-dioxo-2H-iso-indole ethanedioate (1:1). m.p. 159 °C.

1,3-dihydro-2-[N-(2,3-dihydro-1,4-dioxino[2,3-b]pyridin-3-ylmethyl)-3-aminopropyl]-1,3-dioxo-2H-isoindole. m.p. 114 °C.

8-[N-(2,3-dihydro-1,4-dioxino[2,3-b]pyridin-3-ylmethyl)-4-aminobutyl]-8-azaspiro[4.5]decane-7,9-dione hydrochloride (1:2). IR (cm⁻¹): 1710, 1650.

8-[N-(2,3-dihydro-1,4-dioxino[2,3-b]pyridin-3-ylmethyl)-4-aminobutyl]-8-azaspiro[4.5]decane-7,9-dione (E)-2-butenedioate (1:1). m.p. 181 °C.

1,3-dihydro-2-[N-(2,3-dihydro-1,4-dioxino[2,3-b]pyridin-3-ylmethyl)-2-aminoethyl]-1,3-dioxo-2H-isoindole. m.p. 146-148 °C.

2-[N-(2,3-dihydro-1,4-dioxino[2,3-b]pyridin-3-ylmethyl)-4-aminobutyl]-3-oxo-2H-1,2-benzisothiazol-1,1-dioxide (E)-2-butenedioate (1:1). syrup; IR (cm⁻¹): 1715, 1660.

8-[N-(2,3-dihydro-1,4-dioxino[2,3-b]pyridin-3-ylmethyl)-N-methyl-4-aminobutyl]-8-azaspiro[4.5]decane-7,9-dione ethanedioate (1:1). m.p. 153 °C.

1-[N-(2,3-dihydro-1,4-dioxino[2,3-b]pyridin-3-ylmethyl)-4-aminobutyl]-pyrrolidin-2-one (E)-2-butenedioate (1:1). m.p. 147 °C.

1-[N-(2,3-dihydro-1,4-dioxino[2,3-b]pyridin-3-ylmethyl)-4-aminobutyl]-piperidin-2-one (E)-2-butenedioate (1:1). m.p. 142 °C.

1-[N-(2,3-dihydro-1,4-dioxino[2,3-b]pyridin-3-ylmethyl)-4-aminobutyl]-hexahydro-2H-azepin-2-one (E)-2-butene-dioate (1:1). m.p. 124 °C.

### Example 3

The amine of Example 1d can also be prepared from 2-chloro-3-(oxiranylmethoxy)pyridine (Example 1a) by refluxing 1.07 g of the oxirane derivative and 525 mg of sodium azide in 23 ml of dioxane during 7 h. After evaporation of the solvent, extraction with dichloromethane and drying over magnesium sulfate, 1,17 g (88%) of 3-azido-1-(2-chloro-3-pyridinyloxy)-2-propanol was obtained after silica column chromatography (diethyl ether-petroleum ether 2:1). 1.06 g Of this compound dissolved in 15 ml of 1,2-dimethoxymethane was added portionwise under an atmosphere of nitrogen, to 240 mg of sodium hydride 50%, which were washed with a minimum amount of tetrahydrofuran. The reaction mixture was stirred for 24 h at 80 °C, cooled, hydrolized with water, extracted with dichloromethane, and purified by silica chromatography (diethyl ether-petroleum ether 2:1), to give 550 mg (62%) of 3-(2,3-dihydro-1,4-dioxino[2,3-b]pyridine)methanazide. 500 mg Of this azide was hydrogenated in a Parr apparatus in 16 ml of ethanol with 80 mg of Lindlar catalyst under a pressure of hydrogen (30 PSI). After 4 h the solution was filtered and purified by silica chromatography (dichloromethane-methanol 9:1) to give 420 mg (98%) of 3-(2,3-dihydro-1,4-dioxino[2,3-b]pyridine)methanamine.

### Example 4

The amine of Example 1d can also be prepared from 2-chloro-3-(oxiranylmethoxy)pyridine (Example 1a) by adding 1 g of the oxirane derivative to a suspension of 34 g of aluminum oxide in 50 ml of tetrahydrofuran and 5.82 g of benzylalcohol, and stirring the mixture during 24 h. Methanol (20 ml) was added and the mixture was stirred during 2.5 h. After filtration, evaporation of the solvent and purification by silica chromatography (diethyl ether-petroleum ether 2:1), 1,33 g (83%) of 1-(2-chloro-3-pyridinyloxy)-3-(phenylmethyloxy)-2-propanol were obtained. In a manner, similar as described in Example 1c, this derivative was cyclized, and then hydrogenated, in a way as described in Example 3, to give 3-(2,3-dihydro-1,4-dioxino[2,3-b]pyridine)methanol. To a suspension of 81 mg of this alcohol and 382 mg of triphenylphosphine, was added under an atmosphere of argon a complex of zinc azide in pyridine (141 mg). After stirring 0.286 g of diisopropyl azodicarboxylate were added portionwise and the mixture was stirred during 30 min. After purification by silica chromatography (petroleum ether-ethyl acetate 7:3→1:1), 75 % of 3-(2,3-dihydro-1,4-dioxino[2,3-b]pyridine)methanazide was obtained, which was hydrogenated in a Parr apparatus as described in Example 3.

### Example 5

To a suspension containing 0.5 g (R)- or (S)- glycidol in 10 ml of tetrahydrofuran, were added 1.14 g of 2-chloro-3-pyridinol and 2.12 g of triphenylphosphine. The mixture is stirred under argon for 15 min at room temperature, after which 1.6 ml of diisopropyl azodicarboxylate were added at 0 °C. The solution was stood for 1 h at ambient temperature. The mixture was washed with 0.5% aqueous sodium carbonate, extracted with dichloromethane, dried over magnesium sulfate and chromatographed over silica to obtain 82% yield of 2-chloro-3-(oxiranylmethoxy)pyridine, m.p. 36 °C.
1.07 g Of 2-chloro-3-(oxiranylmethoxy)pyridine were dissolved in 23 ml of dioxane, and 525 mg of sodium azide in 6 ml of water were added. The reaction mixture is refluxed for 7 h, after which the solvents were removed, 15 ml of water were added to the residue, and the organic material was extracted with dichloromethane, dried over magnesium sulfate, and chromatographed over silica to give 88% yield of 1-(2-chloro-3-pyridinyloxy)-3-azido-2-propanol.
To a suspension of 0.75 g of sodium hydride, which was washed with a small amount of tetrahydrofuran, were added 3.23 g of 1-(2-chloro-3-pyridinyloxy)-3-azido-2-propanol in 25 ml of ethylene glycol dimethyl ether. The reaction mixture is heated for one night at 80 °C, hydrolysed, extracted, and purified by chromatography over silica to give 62% yield of 2,3-dihydro-3-azido-methyl-1,4-dioxino[2,3-b]pyridine, m.p. 64 °C.
0.54 g of this azide in 17 ml of ethanol is stirred with 86 mg of Lindlar catalyst in a Parr apparatus at a hydrogen pressure of 207 kPa. After 24 h the catalyst is removed by filtration and washed with ethanol. The solvent is removed and the residue purified by chromatography over silica to give 98% yield of (R) or (S) 3-(2,3-dihydro-1,4-dioxino[2,3-b]pyridinyl)methanamine. These products were further processed according to the method of example 1 to obtain:
(R)(+) 8-[N-(2,3-dihydro-1,4-dioxino[2,3-b]pyridin-3-yl-methyl)-4-aminobutyl]-8-azaspiro[4.5]decane-7,9-dione; [α]D²⁰ = +18° (c=1.1, chloroform).

(S)(-) 8-[N-(2,3-dihydro-1,4-dioxino[2,3-b]pyridin-3-yl-methyl)-4-aminobutyl]-8-azaspiro[4.5]decane-7,9-dione; [α]D²⁰ = -18° (c=1.1, chloroform).

(R)(+) 8-[N-(2,3-dihydro-1,4-dioxino[2,3-b]pyridin-3-yl-methyl)-4-aminobutyl]-8-azaspiro[4.5]decane-7,9-dione (E)-2-butenedioate; m.p. 189 °C; [α]D²⁰ = +34° (c=1.0, methanol).

(S)(-) 8-[N-(2,3-dihydro-1,4-dioxino[2,3-b]pyridin-3-yl-methyl)-4-aminobutyl]-8-azaspiro[4.5]decane-7,9-dione (E)-2-butenedioate; m.p. 174 °C; [α]D²⁰ = -37° (c=1.0, methanol).

## Claims

1. A 1,4-dioxino[2,3-b]pyridine derivative having formula I 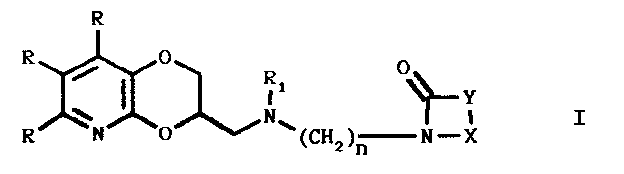 wherein each of the groups R is independently selected from hydrogen, hydroxy, lower alkyl, lower alkoxy, CF₃, and halogen;
R₁ is hydrogen or lower alkyl;
n is 2, 3, or 4;
X is CH₂, C=O, or SO₂; and
Y is substituted or unsubstituted 1,2-phenylene, -(CH₂)₄-, -CH₂CR₂R₃-, or -CH₂CR₂R₃CH₂-, in which R₂ and R₃ are independently hydrogen or lower alkyl, or together 1,4-butanediyl or 1,5-pentanediyl; or a pharmaceutically acceptable salt thereof.

2. The 1,4-dioxino[2,3-b]pyridine derivative of claim 1, wherein R is hydrogen; or a pharmaceutically acceptable salt thereof.

3. The 1,4-dioxino[2,3-b]pyridine derivative of claim 1 or 2, wherein X is C=O; or a pharmaceutically acceptable salt thereof.

4. The 1,4-dioxino[2,3-b]pyridine derivative of claim 2, wherein R₁ is hydrogen, n is 4, X is C=O, Y is - CH₂CR₂R₃CH₂-, and R₂ and R₃ are together 1,4-butanediyl; or pharmaceutically acceptable salts thereof.

5. The levorotatory enantiomer of the 1,4-dioxino[2,3-b]pyridine derivative of claim 2, wherein R₁ is hydrogen, n is 4, X is C=O, Y is -CH₂CR₂R₃CH₂-, and R₂ and R₃ are together 1,4-butanediyl; or pharmaceutically acceptable salts thereof.

6. The 1,4-dioxino[2,3-b]pyridine derivative of any one of claims 1-5 for use in therapy.

7. A process for the preparation of the 1,4-dioxino-[2,3-b]pyridine derivative of claim 1, characterized in that
a) a pyridine derivative having the formula II 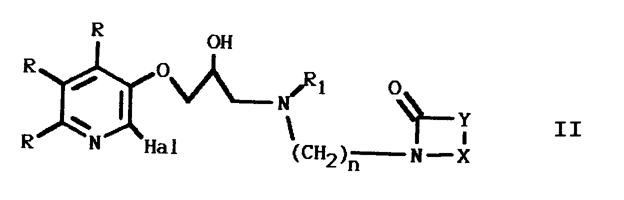 wherein R, R₁, n, X and Y have the previously given meanings, and Hal is Cl, Br, or I, is cyclized, or
b) an amine having formula III 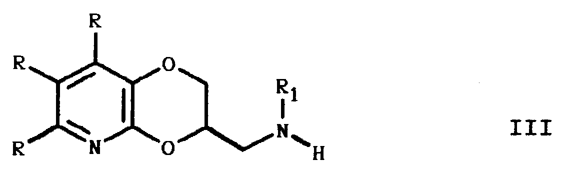 wherein R and R₁ have the previously given meanings, is condensed with a compound having formula IV 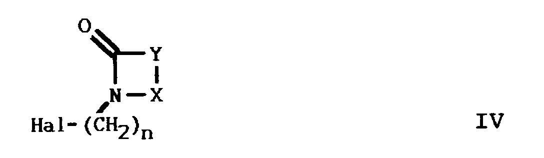 wherein Hal, n, X and Y have the previously given meanings, or
c) a halide having formula V 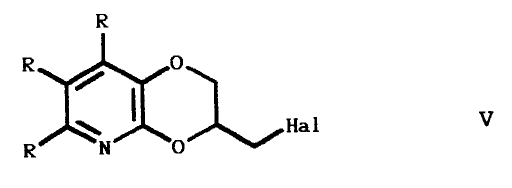 wherein R and Hal have the previously given meanings, is condensed with an amine having formula VI 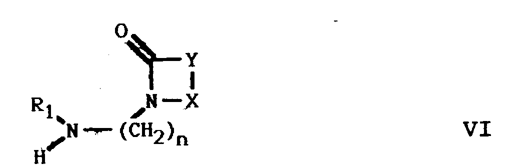 wherein R₁, n, X and Y have the previously given meanings, after which the compound obtained may be separated into its enantiomers and/or converted into a pharmaceutically acceptable salt.

8. A pharmaceutical composition containing at least one of the 1,4-dioxino[2,3-b]pyridine derivatives of any one of claims 1-6, admixed with pharmaceutically acceptable auxiliaries.

9. A use of the 1,4-dioxino[2,3-b]pyridine derivative of any one of claims 1-6 for the manufacture of a medicament having serotonergic activity.
